# EUROPEAN PATENT APPLICATION

(11) **EP 4 620 550 A1**
(43) Date of publication of application: **24.09.2025**
(21) Application number: 23891250.5
(22) Date of filing: 12.10.2023
(51) Int. Cl.: B01D 29/01, C12M 1/28, C12N 1/00

(54) **WELL ARRAY FILTER, PARTICLE ALIGNMENT DEVICE, AND PARTICLE CAPTURING METHOD**

(30) Priority: 14.11.2022 JP 2022181801
(71) Applicant: Tokyo Ohka Kogyo Co., Ltd., Kawasaki-shi, Kanagawa 211-0012 (JP)
(72) Inventor: OHSAKA, Takashi, Kawasaki-shi, Kanagawa 211-0012 (JP); TAKAHASHI, Anna, Kawasaki-shi, Kanagawa 211-0012 (JP); MORITA, Kaho, Kawasaki-shi, Kanagawa 211-0012 (JP); MUROTA, Atsushi, Kawasaki-shi, Kanagawa 211-0012 (JP); NAKAMURA, Akimasa, Kawasaki-shi, Kanagawa 211-0012 (JP)
(74) Representative: Plasseraud IP
(86) International application number: PCT/JP2023/037086
(87) International publication number: WO 2024/106092

(57) **Abstract**

In this well array filter (1), a plurality of wells (3) are formed in a filter body (2) and openings of the wells (3) have a polygonal shape or a polygonal shape with rounded corners. An equivalent circle diameter of the openings of the wells (3) is 5 µm or more and 200 µm or less and at least one through hole (8) is formed in a well bottom portion (4). Well partition walls (6) have a maximum thickness of 1 µm or more and 20 µm or less, a ratio of a total opening area of the wells (3) to an area of a well formation region is 40% or more, and the wells (3) have a depth which is twice or more of the thickness of the well partition walls (6).

## Description

### TECHNICAL FIELD

The present invention relates to a well array filter, a particle alignment device, and a particle capturing method in which particles such as cells and beads are captured.

Priority is claimed on Japanese Patent Application No. 2022-181801, filed November 14, 2022, the content of which is incorporated herein by reference.

### BACKGROUND ART

In recent years, particularly in the field of drug discovery, the target of cell analysis is subdivided from a cell group level to a single cell level, a cell screening device (particle alignment device) is used to capture cells one by one in a large number of fine wells of a well array filter, a screening test is then performed on a large number of cells at once, and cells having desired characteristics are selected. As cell screening methods, for example, a method in which cells captured in a large number of wells are brought into contact with a liquid in which a reagent such as a catcher that binds to a specific antibody is dispersed, and cells that have secreted a secretion bound to the catcher are found, and collected from the wells is used.

The particle alignment device disclosed in Patent Document 1 includes a well array filter made of a resist, and in the well array filter, a large number of wells, each having one through-hole in the bottom portion, are formed in an array. The opening shape of each well is a circle or ellipse, resembling a cell. In examples of Patent Document 1, a well array filter in which the opening diameter of the well is 100 µm, the depth of the well is 30 to 100 µm, and the opening diameter of the through-hole is 2 to 5 µm is disclosed.

The particle alignment device disclosed in Patent Document 2 includes a well array filter made of a silicon wafer, and in the well array filter, a large number of wells are formed in an array by etching, and a SiNx film having one through-hole formed therein is provided in the bottom portion of each well. The opening shape of each well is a circle. This well array filter is made of a silicon wafer, and in examples of Patent Document 2, the thickness of the filter body is 380 µm, the opening diameter of the well is about 100 µm, and the distance between the centers of the wells is about 150 µm.

The particle alignment device disclosed in Patent Document 3 includes a well array filter for secretion assay, which is formed of two layers: a well substrate having a through-hole in the bottom portion for storing cells and a substrate for storing cells for detecting secretions from the cells. The well substrate is made of a resist. In examples of Patent Document 3, the wells are circular and have an opening diameter of 12 µm, which is a size close to that of cells, and the distance between the centers of the wells is about 100 µm.

### CITATION LIST

### Patent Document

Patent Document 1: US Patent No. 10370630
Patent Document 2: US Patent No. 9638636
Patent Document 3: Japanese Unexamined Patent Application, First Publication No. 2019-213566

### SUMMARY OF INVENTION

### Technical Problem

In all of the well array filters described in Patent Documents 1 to 3, distances between wells are large. Thus, particles such as cells and beads frequently do not enter the wells and remain on partition walls between the wells in many cases. Furthermore, since the distance between the wells is large, the wells cannot be arranged at high density and an insufficient well array density is provided for screening a number of cells or the like at once.

In addition, since the density of the well array is low, when through holes in a well bottom portion are formed to have a size in which cells or the like can be reliably captured, that is, cells or the like cannot pass therethrough, a proportion of a total opening area of the through holes included in an area of the well array filter is small, and thus there are problems that it is difficult to cause a sufficient amount of liquid sample to pass through the well array filter merely by a pipetting operation.

Furthermore, since an opening shape of the well has a circular shape or an elliptical shape, when images of cells or the like captured in the well are automatically analyzed, the shapes of the cells and the wells are similar, and thus there is a problem that it is difficult to perform image analysis.

In addition, in all Patent Documents 1 to 3, the well partition walls are thick, and in Patent Document 2, the well partition wall is made of hard silicon. Thus, the filters have poor flexibility, and at the time of collecting cells or the like from the well using a thin glass capillary, the tip of the capillary is likely to hit the well partition wall and be damaged, and thus there is also a problem that the operation is difficult to perform.

The present invention has been made in consideration of the above circumstances, and an object of the present invention is to provide a well array filter, a particle alignment device and a particle capturing method in which capturing particles such as cells one by one in a well is easily performed, screening of a number of particles is easily performed, and image analysis is also easily performed automatically.

### Solution to Problem

[Aspect 1] A well array filter in an aspect according to the present invention includes a flat filter body, the filter body has a plurality of wells each opening in a well formation region on an upper surface of the filter body, the wells which are adjacent to each other are separated by a well partition wall, a well bottom portion is formed at a lower end of each of the wells, an opening of each of the wells has a polygonal shape or a polygonal shape with rounded corners, an equivalent circle diameter of the opening of each of the wells is 5 µm or more and 200 µm or less, at least one through hole is formed in the well bottom portion to reach a lower surface of the filter body, a maximum thickness in a horizontal direction of each of the well partition walls is 1 µm or more and 20 µm or less, a ratio of a total opening area of the well to an area of the well formation region is 40% or more, and a depth of each of the wells is at least twice the maximum thickness in the horizontal direction of the well partition wall..

According to the well array filter in Aspect 1, since the well partition walls between the wells are sufficiently thin, particles such as cells and beads are less likely to remain on the well partition walls. In addition, by appropriately setting the opening diameter, a percentage at which a plurality of particles enter one of the wells can be reduced and the particles can be stably captured in the wells which have sufficient depth compared to the opening diameter. Furthermore, since the wells have a polygonal shape, the wells are easily distinguished from particles during automated image analysis, and furthermore, since the wells can be arranged at high density, it is possible to screen a number of particles at once.

[Aspect 2] In Aspect 1 described above, a maximum inscribed circle diameter of the through hole may be 4 µm or less.

In this case, it is possible to effectively prevent particles such as cells or beads from passing through the through holes.

[Aspect 3] In Aspect 1 or 2 described above, a ratio of a total opening area of the through holes to the area of the well formation region may be 0.5% or more and 3.5% or less.

In this case, since the total opening area of the through hole is appropriate, passing the liquid through the well array filter through pipetting operation is easily performed.

[Aspect 4] In any one of Aspects 1 to 3 described above, the polygonal shape may be any of a triangular shape, a quadrangular shape, and a sexangular shape.

In this case, it is possible to arrange the wells in a closely packed manner on the filter body by making the well shape have any of a triangular shape, a quadrangular shape, or a sexangular shape.

[Aspect 5] In any one of Aspects 1 to 4 described above, the well partition wall and the well bottom portion may be formed of different materials and the well bottom portion may be formed of a material stronger than that of the well partition wall.

In this case, since the well bottom portion is formed of a material stronger than that of the well partition walls, the strength of the entire well array filter is increased while shrinkage during molding of the well wall portion is mitigated, thereby reducing warping and distortion of the well array filter.

[Aspect 6] In any one of Aspects 1 to 5 described above, the well partition wall and the well bottom portion may be formed of different materials, each of the well bottom portions may be divided in a tile shape in which it corresponds to a shape of an opening of the well, gaps may be formed between neighboring well bottom portions, and a material forming the well partition walls is filled and solidified in the gaps..

According to this structure, after the well bottom portion is divided into tile shapes in which gaps are formed therebetween, the well partition walls can be formed and the material forming the well partition walls can be inserted into the gaps and solidified. Thus, warping and distortion which occur in the well array filter due to solidification shrinkage can be reduced compared to when the well bottom portion is formed as a single plate.

[Aspect 7] In any one of Aspects 1 to 6 described above, at least an inner surface of the well may be coated with a polymer which inhibits adhesion of cells. The polymer may be, for example, an MPC polymer, PEG, PVA, PMEA, and mixtures thereof.

In this case, the polymer with which the inner surface of the well is coated inhibits adhesion of the cells, making it easier to remove the cells or particles from the well. All of the inner surface of the well, the upper surfaces of the well partition walls, the inner surfaces of the through holes, and the lower surface of the filter body may be coated with the polymer. In this case, it is possible to suppress problems such as adhesion of cells or the like to the upper surface of the well partition walls and the lower surface of the filter body.

[Aspect 8] In any one of Aspects 1 to 7 described above, a thickness of the well bottom portion may be 1 nm or more and 2 µm or less.

In this case, since the well bottom portion is thin, observing the captured object in the well through the well bottom portion using an inverted microscope is easily performed. In addition, since the thin portion emits less autofluorescence, the adverse effects of autofluorescence on observation can also be reduced.

[Aspect 9] A particle alignment device of Aspect 9 of the present invention includes the well array filter according to any one of Aspects 1 to 8; and a device body that supports the well array filter and has a sample flow path extending from a well opening side of the well array filter through the through holes toward bottom sides of the wells.

According to the particle alignment device according to Aspect 9, since the well partition walls between the wells are thin, particles such as cells and beads are less likely to remain on the well partition walls. In addition, since the wells have a polygonal shape, distinguishing between particles and wells during automated image analysis is easily performed and the wells are sufficiently deep compared to the opening diameter to stably trap particles one by one. Furthermore, since the wells can be arranged at high density, it has the advantage that a large number of particles can be screened at once.

[Aspect 10] A particle capturing method according to Aspect 10 of the present invention includes a step of passing a liquid including an organic solvent which does not dissolve or degrade the well array filter and the device body through the sample flow path of the particle alignment device according to Aspect 9; a step of passing an aqueous solution through the sample flow path and replacing the liquid including the organic solvent with the aqueous solution; and a step of passing an aqueous solution including particles to be captured in the wells through the sample flow path having the replaced aqueous solution and capturing the particles in the wells. Although the liquid including the organic solvent is not limited, for example, liquids such as ethyl alcohol or aqueous solutions including organic solvents such as about 30 to 80% ethyl alcohol, preferably a 70% ethyl alcohol aqueous solution, and the like can be used.

According to the particle capturing method relating to Aspect 10, by passing the liquid including the organic solvent through the sample flow path, and then after replacing the liquid in the sample flow path with an aqueous solution, passing an aqueous solution including particles such as cells, beads having components or secretions of cells attached thereto, or beads for attaching components or secretion of cells, the liquid passage resistance of the well array filter is reduced and air bubbles are less likely to remain in the wells, making it easy to capture the cells or particles one by one in each well.

### Advantageous Effects of Invention

As described above, according to the well array filter of the present invention, since the well partition walls between the wells are sufficiently thin, particles such as cells and beads are less likely to remain on the well partition walls. In addition, by appropriately setting the opening diameter, a percentage at which a plurality of particles enter one of the wells can be reduced and the particles can be stably captured in the wells which have a sufficient depth compared to the opening diameter. Furthermore, since the wells have a polygonal shape, the wells are easily distinguished from particles during automated image analysis, and furthermore, since the wells can be arranged at high density, excellent effects in which a number of particles can be screened at once are achieved.

### BRIEF DESCRIPTION OF DRAWINGS

[FIG. 1] FIG. 1 is an enlarged plan view of a well array filter according to a first embodiment of the present invention.
[FIG. 2] FIG. 2 is an enlarged cross-sectional view of the well array filter of the embodiment.
[FIG. 3] FIG. 3 is an enlarged plan view of the well of a well array filter of the embodiment.
[FIG. 4] FIG. 4 is an enlarged plan view of a well of a well array filter of a second embodiment.
[FIG. 5] FIG. 5 is an enlarged plan view of a well of a well array filter of a third embodiment.
[FIG. 6] FIG. 6 is an enlarged plan view of a well of a well array filter of a fourth embodiment.
[FIG. 7] FIG. 7 is an enlarged plan view of a well of a well array filter of a fifth embodiment.
[FIG. 8] FIG. 8 is an enlarged plan view of a well of the well array filter of a sixth embodiment.
[FIG. 9] FIG. 9 is an enlarged plan view of a well array filter of a seventh embodiment.
[FIG. 10] FIG. 10 is an enlarged plan view of a well array filter of an eighth embodiment.
[FIG. 11] FIG. 11(a) to FIG. 11(f) are enlarged cross-sectional views showing a method of producing a well array filter according to an embodiment of the present invention.
[FIG. 12] FIG. 12 is a plan view showing a particle alignment device of an embodiment of the present invention.
[FIG. 13] FIG. 13 is a longitudinal cross-sectional view showing a particle alignment device of the embodiment.
[FIG. 14] FIG. 14 is a graph showing an effect of an example of the present invention.
[FIG. 15] FIG. 15 is a graph showing an effect of an example of the present invention.
[FIG. 16] FIG. 16 is a graph showing an effect of an example of the present invention.
[FIG. 17] FIG. 17 is a photograph showing an effect of an example of the present invention.

### DESCRIPTION OF EMBODIMENTS

Embodiments of the present invention will be described below with reference to the drawings.

### [First embodiment]

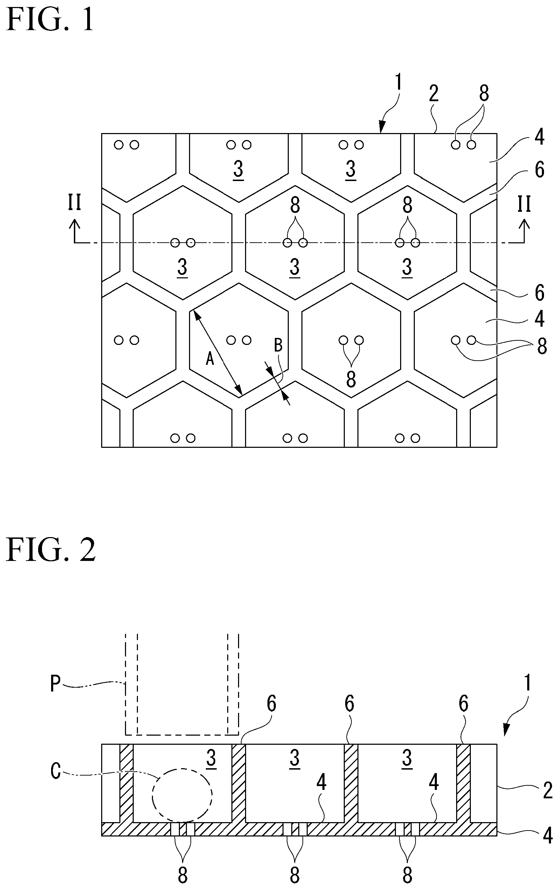

FIGS. 1 and 2 are an enlarged plan view and an enlarged cross-sectional view showing a first embodiment of a well array filter. The well array filter 1 has a flat and plate shaped filter body 2 which has a constant thickness, and the filter body 2 has a number of wells 3 formed therein that open into a well formation region on an upper surface of the filter body 2. The well formation region refers to a region of the filter body 2 which is used as a filter and the filter body 2 may have, around the well formation region, a support region or the like which supports the filter and in which no well is formed.

The wells 3 adjacent to each other are separated using well partition walls 6. As shown in FIG. 2, a flat well bottom portions 4 is formed at a lower end of each of the wells 3 and closes the lower end of the well 3. An opening of the well 3 has a polygonal shape or a polygonal shape with rounded corners. In the embodiment, the well 3 has a regular hexagonal shape and each of the wells 3 and each of the well partition walls 6 are arranged in a honeycomb pattern. At least the well bottom portion 4 is made of a transparent material so that particles such as cells C in the wells 3 can be observed through the well bottom portion 4 using an inverted microscope. The well partition walls 6 may also each be made of a transparent material, but may also be made of an opaque material.

The equivalent circle diameter of the opening of the well 3 is 5 µm or more and 200 µm or less. The expression "equivalent circle diameter" refers to a diameter of a circle having an area that is the same as that of the openings of the wells 3. If the equivalent circle diameter of the opening of the well 3 is 5 µm or more, it becomes possible to capture particles such as cells or beads. In addition, if the equivalent circle diameter of the opening of the well 3 is 200 µm or less, it becomes easy to capture one or an appropriate fixed number of particles such as cells or beads in each of the wells 3. The equivalent circle diameter of the opening of the well 3 may be more preferably 10 µm or more and 100 µm or less and further preferably 15 µm or more and 60 µm or less so that the cells can be captured one by one using the well 3.

The beads are particles made of a resin or the like, and the component or the secretion of the cells can be attached thereon for analysis. In addition, the beads obtained by releasing the component of the cells outside of the well 3, attaching the component to the beads, and capturing the beads having the components of the cells or the secretion attached thereto may be provided for analysis or the beads obtained by introducing the cells and the beads into the well 3, breaking the cells, and attaching the component of the cells or the secretion in the well 3 may be provided in some cases. Furthermore, the beads which become magnetized may be used and the beads may be attracted to a magnet disposed below the well array filter 1 and captured in the well 3 in some cases.

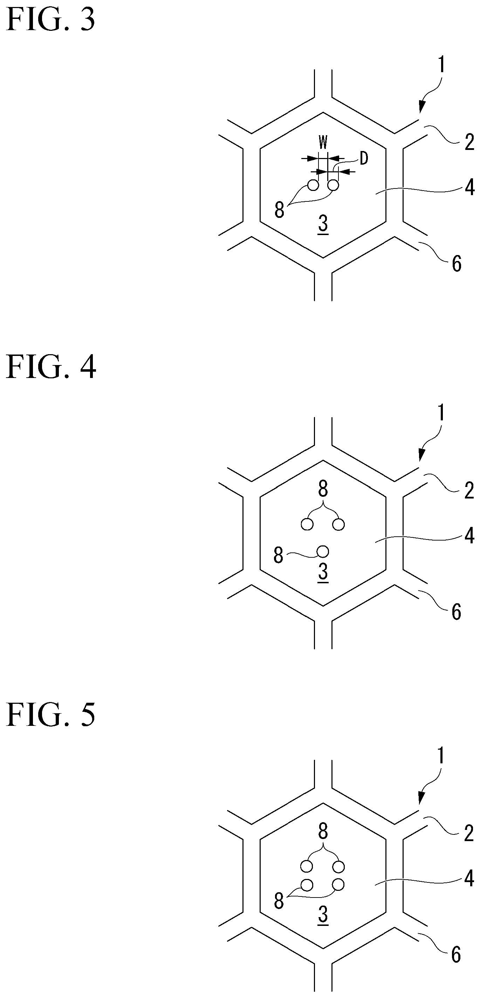

At least one through hole 8 reaching a lower surface of the filter body 2 is formed in each of the well bottom portions 4 one by one. In the embodiment, the number of through holes 8 is two for each of the wells 3, and as shown in FIG. 3, the through holes 8 are formed at a center of the well bottom portion 4 with an interval W therebetween so that they vertically pass through the well bottom portion 4. Although the number of through holes 8 is not limited and may be one, three, or four or more in the present invention, when only one through hole 8 is formed in a center, if a diameter D of the through hole 8 is made large to some extent to ensure a sufficient flow rate, the cells C are likely to pass through the through hole 8 while deforming. When a plurality of through holes 8 are formed in the well bottom portion 4, there is an advantage that the cells C are unlikely to pass through the through holes 8 even when a sufficient flow rate has been ensured and at least some of the through holes 8 are formed in positions off-center from the well bottom portion 4. Thus, it is unlikely that all of the through holes 8 are blocked by the cells C and it is easy to allow a liquid in the wells 3 to circulate appropriately through the through holes 8 even after the cells C are captured.

A maximum thickness B (refer to FIG. 1) in a horizontal direction of the well partition walls 6 is set to be 1 µm or more and 20 µm or less. By setting the maximum thickness B to 1 µm or more, a strength of the well partition walls 6 can be adjusted appropriately and the well partition walls 6 can be prevented from collapsing, for example, when a pipette P is applied to the upper surface of the well partition walls 6 to suck up cells C. Furthermore, by making the maximum thickness B 20 µm or less, particles to be captured such as cells or beads can be prevented from remaining on the well partition walls 6 and a density of the wells 3 can be increased. Moreover, when the pipette P is applied to the well partition walls 6, appropriate flexibility can be imparted to the well partition walls 6 and damage to the distal end of the pipette P can be suppressed. The maximum thickness B of the well partition walls 6 may be preferably 2 µm or more and 20 µm or less, and more preferably 2 µm or more and 12 µm or less.

A ratio of the total opening area of the wells 3 to an area of the well formation region is 40% or more. If this ratio is 40% or more, a ratio of particles captured in the wells 3 of particles such as cells supplied to the well array filter 1, that is, a particle capture rate, can be sufficiently increased. A ratio of the total opening area of the wells 3 to an area of the well formation region may be preferably 40% or more and 95% or less, and more preferably 40% or more and 75% or less. The expression of a ratio which is high means that the well partition walls 6 are thin.

A depth of the wells 3 is set to be twice or more the maximum thickness B in the horizontal direction of the well partition walls 6. If the depth of the wells 3 is twice or more the thickness B of the well partition walls 6, particles such as cells C captured in the wells 3 can be prevented from escaping from the wells 3 due to disturbances or the like in flow of the liquid. Thus, once captured, the particles can be stably retained in the wells 3. More preferably, the depth of the wells 3 may be 2 times or more and 40 times or less the thickness B of the well partition walls 6, and further preferably, 2 times or more and 15 times or less.

Although a shape of the through holes 8 when viewed in a plan view is a circular shape in the embodiment, the present invention is not limited to a circular shape and may be any shape such as an elliptic shape, a triangular shape, a quadrangular shape, a pentagonal shape, a hexagonal shape, or other polygonal shape, a polygon with rounded corners, a rectangle, a star, a slit, a dumbbell shape, an H-shape, or any other irregular shape.

A maximum inscribed circle diameter of the through holes 8 is not limited in the present invention, but is preferably 4 µm or less. The maximum inscribed circle diameter refers to a diameter of an inscribed circle when the largest inscribed circle is drawn in one of the through holes 8. If the maximum inscribed circle diameter exceeds 4 µm, particles such as cells and beads to be captured are likely to slip through. Preferably, the maximum inscribed circle diameter is 0.1 µm or more and 4 µm or less, and more preferably, 0.5 µm or more and 3.5 µm or less.

A ratio of the total opening area of the through holes 8 to an area of the well formation region is not limited in the present invention, but is preferably 0.5% or more and 3.5% or less. The total opening area of the through holes 8 is a total value of opening areas (=flow path cross-section area) of all of the through holes 8 formed in the well formation region. If the proportion of the total opening area of the through holes 8 is 0.5% or more, a resistance of a flow of the liquid passing through the through holes 8 is appropriately reduced. Thus, the liquid easily passes through the well array filter 1 through the operation of the pipette P. If the ratio of the total opening area of the through holes 8 is 3.5% or less, at the time of passing the liquid through the well array filter 1 through the operation of the pipette P, a liquid sample does not flow too much, providing excellent operability. The ratio of the total opening area of the through holes 8 may be preferably 0.5% or more and 3.5% or less, and more preferably 0.75% or more and 3.2% or less.

If a shape of the wells 3 when viewed in a plan view is a polygonal shape or a polygonal shape with rounded corners, it will be easy to distinguish the particles from the wells 3 during automated image analysis, but among polygonal shapes, it is preferable that the shape be either a triangular shape, a quadrangular shape, or a hexagonal shape as shown in the drawing. If the wells 3 have a triangular, quadrangular, or hexagonal shape, the wells 3 can be regularly arranged in the same shape, thereby increasing an arrangement density of the wells 3. Among them, if the shape is an equilateral triangle, a regular quadrangle, or a regular hexagon, this is preferable because it is possible to increase the arrangement density of the wells 3 with a geometrically simple array.

The well partition walls 6 and the well bottom portion 4 may be formed of the same material as each other, but preferably are formed of different materials and the well bottom portion 4 may be formed of a material stronger than the well partition walls 6. In this case, the strength of the entire well array filter 1 can be increased while shrinkage during molding of the well bottom portion 4 is mitigated, thereby reducing warping and distortion of the well array filter 1. The fact that warping of the well array filter 1 does not occur is important because the entire observation range can be in focus at the time of observing the particles captured in the wells 3 through an inverted microscope.

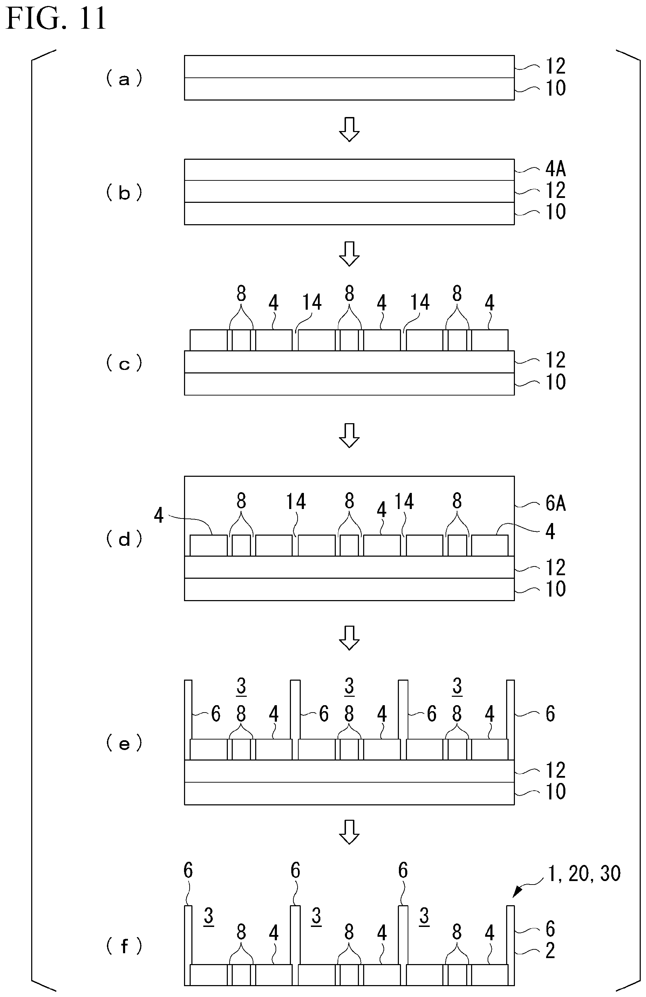

Although the materials of the well partition walls 6 and the well bottom portion 4 are not limited, in order to realize a fine structure thereof, various photoresists may be used. In addition, it is preferable that the well bottom portion 4 be made of a material stronger than the well partition walls 6 by changing a type and a composition of the photoresist and/or changing the exposure conditions. When the well partition walls 6 and the well bottom portion 4 are formed using a photoresist, for example, a partition wall resist 6A (refer to FIG. 11) using which the well partition walls 6 are formed can be formed of a photoresist with a relatively small amount of multi-functional epoxy and a bottom portion resist 4A (refer to FIG. 11) using which the well bottom portion 4 is formed can be formed of a photoresist with a relatively large amount of multi-functional epoxy, making it possible to make the strength of the material of the well bottom portion 4 greater than the strength of the material of the well partition walls 6. In this case, although the autofluorescence of the material of the well partition walls 6 is relatively weak and the autofluorescence of the material of the well bottom portion 4 is relatively strong, the thickness of the well bottom portion 4 is small. Thus, this does not adversely affect observation with an inverted microscope.

Although the method of forming the well bottom portion 4 and the well partition walls 6 is not limited in the present invention, in order to relieve stress in the well array filter 1, preferably, a structure in which the well partition walls 6 and the well bottom portion 4 are formed separately from each other, each of the well bottom portions 4 is divided in a tile shape in which it corresponds to a shape of an opening of the well 3, gaps are formed between neighboring well bottom portions 4 and a material forming the well partition walls 6 enters these gaps and solidifies may be provided. A specific example of the production method will be described later. By forming the well array filter 1 in such a structure in which the well bottom portion 4 is divided into such tiles and the well partition walls 6 enter the gaps between the well bottom portion 4, it is possible to further prevent the well array filter 1 from warping or distortion due to solidification shrinkage of the well bottom portion 4.

Although not essential in the present invention, at least a part of the inner surface of the wells 3 may be pre-coated with a polymer which inhibits adhesion of cells. In this case, the polymer with which the inner surface of the wells 3 is coated suppresses adhesion of the cells C so that the cells C or the particles can be easily removed from the wells 3. As the polymers of this type, for example, a 2-methacryloyloxyethyl phosphorylcholine polymer (MPC polymer), polyethylene glycol (PEG), polyvinyl alcohol (PVA), poly(2-methoxyethyl acrylate) (PMEA), and mixtures thereof can be adopted. In a coating position of the polymer, all of the inner surfaces of the wells 3, the upper surfaces of the well partition walls 6, the inner surfaces of the through holes 8, and the lower surface of the filter body 2 may be coated with the polymer. In this case, problems such as adhesion of cells or the like to the upper surfaces of the well partition walls 6 and the lower surface of the filter body 2 can be suppressed.

Although not limited in the present invention, the thickness of the well bottom portion 4 may be 1 nm or more and 2 µm or less. In this case, since the well bottom portion 4 is sufficiently thin, captured objects such as cells C in wells 3 through the well bottom portion 4 with an inverted microscope are easily observed. In addition, since the well bottom portion 4 is thin, the autofluorescence of the well bottom portion 4 can be suppressed to have a small level and the adverse effects of autofluorescence on observation can also be reduced. The thickness of the well bottom portion 4 may more preferably be 100 nm or more and 2 µm or less, and further preferably be 500 nm or more and 1.5 µm or less.

According to the well array filter 1 having the above-described constitution, since the well partition walls 6 between the wells 3 are sufficiently thin, particles such as cells and beads are less likely to remain on the well partition walls 6. In addition, by appropriately setting the opening diameter, a percentage at which a plurality of particles enter one of the wells 3 can be reduced and the particles can be stably captured in the wells 3 which have a sufficient depth compared to the opening diameter. Furthermore, since the wells 3 have a polygonal shape, the wells 3 are easily distinguished from particles during automated image analysis, and furthermore, since the wells 3 can be arranged at high density, there is an advantage that a number of particles can be screened at once.

### [Second embodiment of well array filter]

FIG. 4 shows a second embodiment of the well array filter 1. In addition, in this example, a total of three through holes 8 are formed in a center of the well bottom portion 4. Each of the through holes 8 may be formed at a vertex position of an equilateral triangle, but may also be formed at each vertex of an isosceles triangle, a right-angled triangle, or a scalene triangle other than an equilateral triangle. When they are arranged in a triangle, it is preferable that the center of the well bottom portion 4 be located inside the triangle, preferably at the center of gravity. Three through holes 8 may be formed on one line segment at equal or unequal intervals. In this case, it is preferable that the center of the well bottom portion 4 be located on a line segment. The other constituent elements of the second embodiment may be the same as those of the first embodiment and the above description is incorporated herein. In this second embodiment, the same effects as in the first embodiment can be obtained.

### [Third embodiment of well array filter]

FIG. 5 shows a third embodiment of the well array filter 1. In addition, in this example, a total of four through holes 8 are formed in a center of the well bottom portion 4. Each of the through holes 8 may be formed at a vertex position of a square, but may also be formed at each vertex of a rectangle, a trapezoid, or a scalene quadrangle other than a square. When the wells are arranged in a quadrangle, it is preferable that the center of the well bottom portion 4 be located inside the quadrangle, preferably at the center of gravity. Furthermore, three through holes 8 may be arranged at the vertex portions of a triangle and a fourth through hole 8 may be disposed inside the triangle or on any of the three sides. In this case, the triangle may be an equilateral triangle, but may also be an isosceles triangle, a right-angled triangle, or a scalene triangle other than an equilateral triangle. When they are arranged in a triangle, it is preferable that the center of the well bottom portion 4 be located inside the triangle, preferably at the center of gravity. Moreover, four through holes 8 may be formed at equal or unequal intervals on one line segment. In this case, it is preferable that the center of the well bottom portion 4 be located on a line segment. The other constituent elements of the third embodiment may be the same as those of the first embodiment and the above description is incorporated herein. In the third embodiment as well, the same effects as those of the first and second embodiments can be obtained.

### [Fourth embodiment of well array filter]

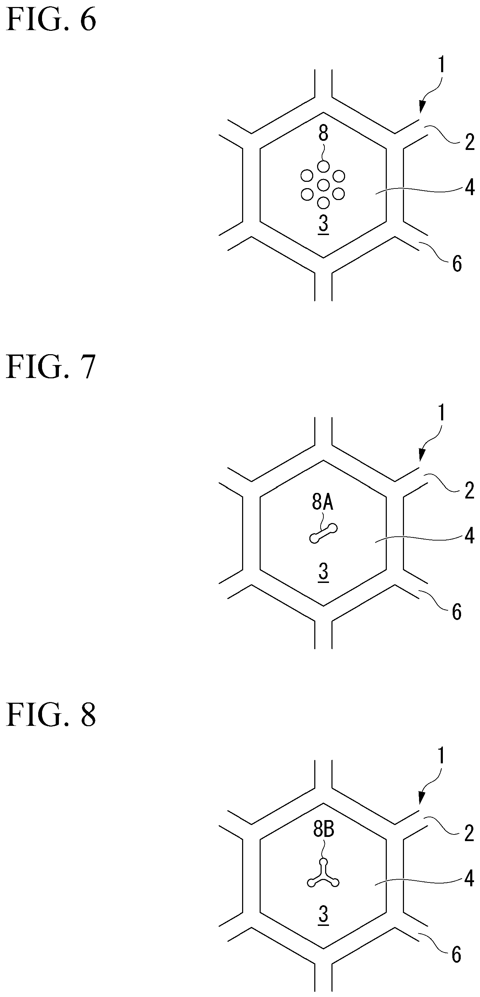

FIG. 6 shows a fourth embodiment of the well array filter 1. In addition, in this example, a total of seven through holes 8 are formed in a center of the well bottom portion 4. In the example of FIG. 6, each of the six through holes 8 is formed at a vertex position of a regular hexagon and the seventh through hole 8 is formed in the center of the regular hexagon. The arrangement is not limited to the arrangement shown in the drawing, in the present invention, N (N≥1) through holes 8 may be formed at vertex positions of a polygon which M vertices (M≥3) and inside the polyangle with M angles or on one or more sides. The other constituent elements of the fourth embodiment may be the same as those of the first embodiment and the above description is incorporated herein. In the fourth embodiment as well, the same effects as in the first to third embodiments can be obtained.

### [Fifth embodiment of well array filter]

FIG. 7 shows a fifth embodiment of the well array filter 1. In addition, in this example, a through hole 8A having a planar shape (a dumbbell shape) in which two circles are connected using a slit is formed in a center of a well bottom portion 4. The through hole 8A may be a simple long hole. The through hole 8A may have a curved line shape or a bent line shape. In the case of the through hole 8A having such a shape, even if a maximum inscribed circle diameter of the through hole 8A is 4 µm or less, a large opening area (flow path cross-section area) of the through hole 8A can be ensured and a flow rate of the through hole 8A can be increased while preventing particles such as cells and beads from passing through the through hole 8A. In this case, it is preferable that the center of the well bottom portion 4 be located on the through hole 8A. The other constituent elements of the fifth embodiment may be the same as those of the first embodiment and the above description is incorporated herein. In the fifth embodiment, the same effects as those of the first to fourth embodiments can be obtained.

### [Sixth embodiment of well array filter]

FIG. 8 shows a sixth embodiment of the well array filter 1. In addition, in this example, a planar-shaped (radial-shaped) through hole 8B which is composed of three or more slits extending radially from the center and circular holes formed at the terminal ends of the slits is formed in a center of the well bottom portion 4. Each slit of the through hole 8B may be a simple long hole. In the case of the through hole 8B having such a shape, even if a maximum inscribed circle diameter of the through hole 8A is 4 µm or less, a large opening area (flow path cross-section area) of the through hole 8B can be ensured and the flow rate of the through hole 8B can be increased while preventing particles such as cells and beads from passing through the through hole 8B. In this case, it is preferable that the center of the well bottom portion 4 be located at the center of the through hole 8B. The other constituent elements of the sixth embodiment may be the same as those of the first embodiment and the above description is incorporated herein. In the sixth embodiment, the same effects as those of the first to fifth embodiments can be obtained.

### [Seventh embodiment of well array filter]

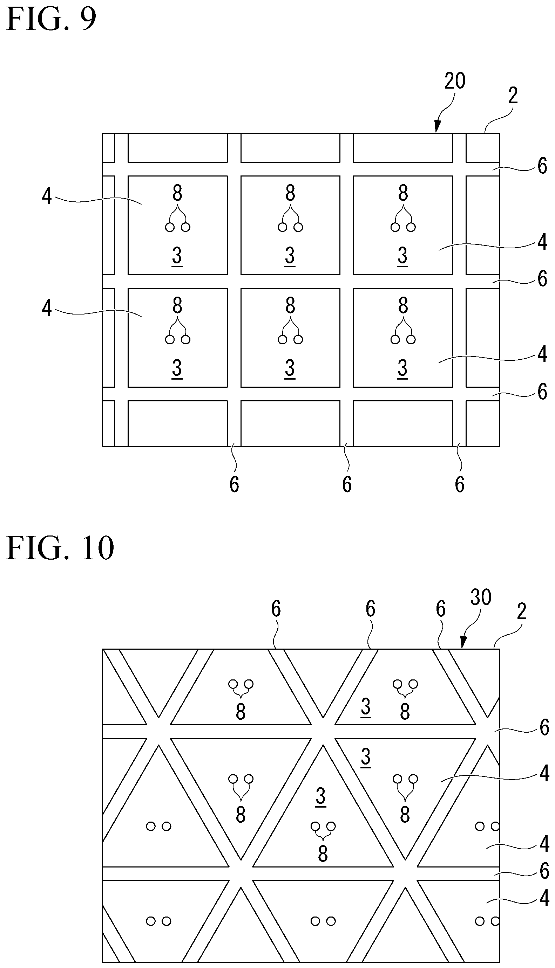

FIG. 9 shows a seventh embodiment 20 of a well array filter. In addition, this example is characterized in that the well partition walls 6 are formed in a regular square grid shape and the well bottom portions 4 are each formed in a square shape. Although the well partition walls 6 are not limited to a regular square grid shape and may be a diagonal grid shape or a rectangular grid shape, it is preferable that the shapes of the individual wells 3 be the same as each other. The wells 3 may be arranged in a staggered fashion in every other row. Through holes 8 may be the same as those of any of the first to sixth embodiments. The other constituent elements of the seventh embodiment may be the same as those of the first embodiment and the above description is incorporated herein. In the seventh embodiment as well, the same effects as those of the first to sixth embodiments can be obtained.

### [Eighth embodiment of well array filter]

FIG. 10 shows an eighth embodiment 30 of a well array filter. In addition, this example is characterized in that the well partition walls 6 are formed in a so-called truss shape in which equilateral triangles are arranged in alternating opposite directions and the well bottom portions 4 are each formed in the shape of an equilateral triangle. Although the well partition walls 6 are not limited to an equilateral triangular shape and may be an isosceles triangular shape, a right-angled triangle, or a scalene triangle, it is preferable that the shapes of the individual wells 3 be the same as each other. Through holes 8 may be the same as those of any of the first to sixth embodiments. The other constituent elements of the eighth embodiment may be the same as those of the first embodiment and the above description is incorporated herein. In the eighth embodiment as well, the same effects as in the first to seventh embodiments can be obtained.

### [Embodiment of method of producing well array filter]

FIGS. 11(a) to 11(f) are cross-sectional views showing an example of a method of producing the well array filters 1, 20, and 30. First, as shown in FIG. 11(a), a sacrificial film 12 which is insoluble in a resist solvent and a developing solution is formed on a substrate 10 such as a Si wafer. The sacrificial film 12 can be formed of, for example, a styrene-based elastomer, dextrin, dextran, or the like.

Subsequently, a state in which, as shown in FIG. 11(b), a negative type bottom portion resist 4A is formed on the sacrificial film 12 to have a constant thickness required as the well bottom portion 4 and the bottom portion resist 4A is exposed to light through photolithography in a predetermined pattern and then developed, and as shown in FIG. 11(c), the well bottom portions 4 having the through holes 8 are arranged in a polyangular tile shape with gaps 14 between these is provided. A width of each of the gaps 14 is set to be slightly smaller than a thickness of the well partition walls 6. Furthermore, the gap 14 between the well bottom portions 4 may have a state in which it is partially bridged through a thin bridge. In this case, the bridge can be used to position the well bottom portions 4 so that the arrangement thereof is not disturbed.

Subsequently, as shown in FIG. 11(d), a partition wall resist 6A with which the well bottom portion 4 is covered is formed to have a required thickness as the well partition wall 6. At this time, for example, it is preferable that the partition wall resist 6A be formed of a photoresist having a relatively small amount of multifunctional epoxy and a bottom portion resist 4A be formed of a photoresist having a relatively large amount of multifunctional epoxy so that the strength of the material of the well bottom portion 4 is relatively higher than the strength of the material of the well partition wall 6. In this case, as a side effect, the autofluorescence of the material of the well partition walls 6 is relatively weak and the autofluorescence of the material of the well bottom portion 4 is relatively strong. Here, since the thickness of the well bottom portion 4 is small, this does not adversely affect observation with an inverted microscope. The partition wall resist 6A is exposed to light in a predetermined pattern through photolithography and then developed to form the well partition walls 6, as shown in FIG. 11(e). Thus, the partition wall resist 6A enters the gap 14 and is insolubilized in the gap 14 through exposure to light, thereby connecting the well bottom portions 4.

Subsequently, the well bottom portion 4 and the well partition walls 6 are heated, for example, to 180 °C to further thermally harden the resist, and then the sacrificial film 12 is dissolved with a solvent which does not alter the resist and the substrate 10 is peeled off from the well bottom portion 4, completing the well array filters 1, 20, and 30 as shown in the first to eighth embodiments.

According to the above production method, the well array filters 1, 20, and 30 having the wells 3 with high shape accuracy can be produced efficiently. Furthermore, according to this production method, the well bottom portion 4 is divided into tile shapes to form the gaps 14 between these, and then the well partition walls 6 are formed and the material forming the well partition walls 6 enters the gap 14 and is solidified, thereby reducing warping and distortion which occurs in the well array filters 1, 20, and 30 due to solidification and shrinkage of the well bottom portion 4.

### [Embodiment of particle alignment device]

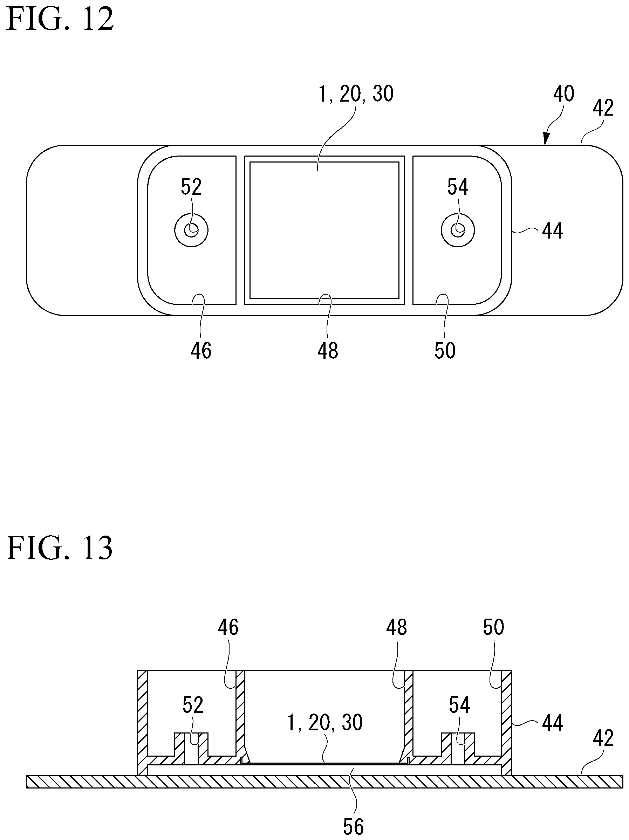

FIGS. 12 and 13 are a plan view and a longitudinal cross-sectional view showing an embodiment 40 of a particle alignment device according to the present invention, respectively. The particle alignment device 40 has a rectangular bottom plate portion 42 and a wall portion 44 formed at a central portion of the bottom plate portion 42. The wall portion 44 has a square frame-shaped peripheral wall portion which stands up from the bottom plate portion 42 and two partition walls which are formed parallel to each other and stand up on the inside of the peripheral wall portion and the inside of the peripheral wall portion is divided into a first chamber 46, a second chamber 48, and a third chamber 50. A cylindrical opening 52 is formed in the bottom portion of the first chamber 46 and cylindrical openings 54 are also formed in the bottom portion of the third chamber 50.

The bottom of the second chamber 48 is open and the well array filters 1, 20, and 30 are fixed to the lower end of the wall portion 44 with a small flow path 56 spaced from the bottom plate portion 42, as shown in FIG. 13. Certain gaps are each provided between the bottom portion of the first chamber 46 and the bottom plate portion 42 and between the bottom portion of the third chamber 50 and the bottom plate portion 42, forming the flow path 56. Thus, by introducing a liquid sample including particles such as cells C or beads in the second chamber 48 and allowing a liquid to flow out from the openings 52 or the openings 54, it is possible to capture particles such as cells C or beads in the wells 3 of the well array filter 1. That is to say, a sample flow path which reaches the second chamber 48, the well array filter 1, and the flow path 56 is formed. Furthermore, a reagent can also be introduced from the openings 52 or the openings 54 to react with particles such as cells C or beads in the second chamber 48.

### [Embodiment of particle capturing method]

An embodiment of a particle capturing method according to the present invention has a step of passing a liquid including an organic solvent which does not dissolve or degrade a well array filter 1 and a device body 40 through a sample flow path passing through the well array filters 1, 20, and 30 of a particle alignment device 40, that is, a sample flow path passing from openings of wells 3 toward bottom surface sides of the wells 3 through through holes 8, a step of passing an aqueous solution through the sample flow path and replacing the liquid including the organic solvent with the aqueous solution, and a step of passing an aqueous solution including particles to be captured in the wells 3 through the sample flow path and capturing the particles in the wells 3.

Specifically, a liquid such as a water-soluble organic solvent having a low interfacial tension such as ethyl alcohol or an aqueous solution including about 30 to 80% organic solvent such as ethyl alcohol, is supplied to a second chamber 48 of the particle alignment device 40 and the liquid including the organic solvent is passed through the well array filters 1, 20, and 30. As the liquid, for example, a 70% ethyl alcohol aqueous solution or the like can also be suitably used. Thus, the through holes 8 are also filled with an organic solvent. Subsequently, an aqueous solution such as a buffer solution such as a phosphate buffered saline (PBS) is added to second chamber 48 and the liquid containing the organic solvent is aspirated from the openings 52 and/or the openings 54. This process is repeatedly performed several times to replace the organic solvent-containing liquid in the second chamber 48, the through holes 8, and the flow path 56 with an aqueous solution of a phosphate buffer solution.

Subsequently, a sample liquid in which particles such as cells C and/or beads are dispersed in a phosphate buffer solution or the like is supplied to the second chamber 48 and the phosphate buffer solution is aspirated from the openings 52 and/or the openings 54, thereby capturing cells C and/or beads one by one or in approximately fixed numbers in each of the wells 3.

Subsequently, a reagent which binds to cells C or beads having secretion of cells attached thereto that are targets is added from the second chamber 48 or the openings 52 and 54, and after marking the target, the target is identified using an inverted microscope and the cells C or the beads that are targets are collected from the wells 3 using a pipette P, a capillary, or the like.

According to the particle capturing methods described above, a liquid including an organic solvent is passed through the sample flow path which passes through the well array filter 1 and replaced with an aqueous solution, and then an aqueous solution including particles such as cells, beads having components or secretion of cells attached thereto, or beads for attaching components or secretion of cells is passed so that the liquid passage resistance is reduced and air bubbles are less likely to remain in the wells 3 and capturing particles such as cells C or beads in each of the wells 3 one by one or a predetermined number by a predetermined number is easily performed. Therefore, it has the advantage that screening of a number of particles can be carried out stably.

Although the embodiments of the present invention have been described above, the present invention is not limited to only these embodiments and additions, deletions, and modifications of constituent elements are possible within the scope described in the claims.

### EXAMPLES

The effects of the present invention will be demonstrated below using examples.

### [Experiment 1]

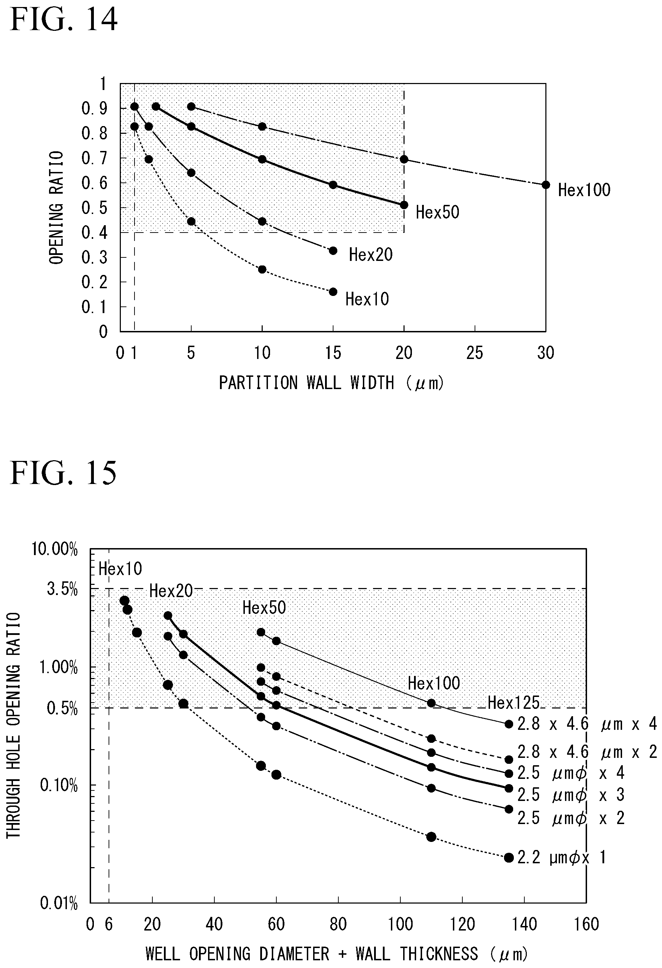

As shown in FIGS. 1 and 2, FIG. 14 is a graph showing an opening ratio of the wells 3 when the well array filter 1 in which the openings of the wells 3 was a regular hexagon was created by variously changing an opening width of the wells 3 and a thickness in the horizontal direction of the well partition walls 6. In FIG. 14, "Hex10" indicates that the wells 3 are regular hexagons with an opening width between two parallel sides of 10 µm, "Hex20" indicates that the opening width is similarly 20 µm, "Hex50" indicates that the opening width is similarly 50 µm, and "Hex100" indicates that the opening width is similarly 100 µm. A partition wall width (µm) on the horizontal axis indicates a thickness (µm) in a horizontal direction of the partition walls of the well partition walls 6. When each of the wells 3 has a regular hexagon and an opening width between two parallel sides is A (µm), the equivalent circle diameter of well 3 is 1.050075×A. An opening ratio on the vertical axis indicates a ratio of the total opening area of the wells 3 to an area of the well formation region. As shown in FIG. 14, when surrounded by the dotted line, it can be seen that the partition width is 1 to 20 µm and the opening ratio is 0.4 (=40%) or more.

### [Experiment 2]

As shown in FIGS. 1 and 2, FIG. 15 is a graph showing the total opening ratio (%) of the through holes 8 when the well array filter 1 in which the openings of the wells 3 had a regular hexagonal shape was created by variously changing an opening width of the wells 3, a thickness of the well partition walls 6, and a dimension, a shape, and the number of the through holes 8. In FIG. 15, "Hex10" to "Hex100" are the same as in FIG. 14 and "Hex125" indicates that the opening width between two parallel sides of the wells 3 is 125 µm. The expression "well opening diameter+wall thickness" in the horizontal axis indicates an addition of an opening width (µm) between the two parallel sides of the wells 3 and a thickness (µm) of the well partition walls 6 in the horizontal direction. A through hole opening ratio (%) in the vertical axis indicates a ratio (%) of the total opening area of the through holes 8 to an area of the well formation region. The expression "2.5 µmΦ×4" indicates that four through holes 8 having a circular diameter of 2.5 µm are formed in each of the wells 3. The expression "2.8 µm×4.6 µm×4" indicates that four rectangular through holes 8 of 2.8 µm×4.6 µm are formed in each of the wells 3. As shown in FIG. 15, when surrounded by the dotted line, it can be seen that the opening width of the wells 3 is 5 µm or more, the thickness of the well partition walls 6 is 1 µm or more, and the through hole opening ratio is 0.5 to 3.5%.

### [Experiment 3]

The well array filters of Examples 1 to 8 and the Comparative Example were actually produced using FIG. 11 and the method described in the embodiment of the method of producing a well array filter. The openings of well 3 had regular hexagons. A partition wall resist 6A is formed of a negative photoresist having a relatively small amount of multifunctional epoxy and a bottom portion resist 4A is formed of a negative photoresist having a relatively large amount of multifunctional epoxy.

Table 1 shows the parameters of the well array filters produced in Examples 1 to 8 and the Comparative Example and "Hex10-20" indicates that the wells 3 have a regular hexagon, a target value of an opening width between the two parallel sides is 10 µm, and a target value of a depth of the wells 3 is 20 µm. The same applies to the others. An "opening diameter (µm)" in a "well shape" indicates an opening diameter between two parallel sides of a regular hexagon, a "partition wall width (µm)" indicates a thickness in the horizontal direction of the well partition walls 6, a "depth (µm)" indicates a depth of the wells 3, an "opening ratio" indicates a ratio (%) of the total opening area of the wells 3 to an area of the well formation region, and a "density (cm⁻²)" indicates the number of wells 3 formed per 1 cm² of the well formation region. The "number of through holes" in a "through hole shape" indicates the number of through holes 8 per well 3, an "opening shape" indicates an opening shape of each of the through holes 8, "2.8" of an "opening dimension" indicates a diameter of a circle, "3.15/5.19" indicates a rectangle of 3.15 µm×5.19 µm having rounded and chamfered corners, and an "opening ratio" indicates the total opening ratio (%) of the through holes 8 to an area of the well formation region.

**TABLE 1**

| | | Well shape | | | | | | Through hole shape | | | | |
|---|---|---|---|---|---|---|---|---|---|---|---|---|
| | | Opening diameter (µm) | Partition wall width (µm) | Depth (µm) | Depth/Partition wall width | Opening ratio | Density (cm⁻²) | Number of through holes | Opening shape | Opening dimension (µm) | | Opening ratio |
| Example 1 | Hex10-20 | 10.2 | 4.80 | 17.7 | 3.69 | 46.2% | 513185 | 1 | Circle | 2.80 | | 3.16% |
| Example 2 | Hex20-25 | 20.3 | 9.62 | 26.4 | 2.74 | 46.0% | 128983 | 2 | Circle | 2.80 | | 1.59% |
| Example 3 | Hex20-50 | 22.1 | 7.00 | 51.2 | 7.31 | 57.7% | 136355 | 2 | Circle | 2.80 | | 1.68% |
| Example 4 | Hex35-50 | 36.0 | 6.00 | 44.3 | 7.38 | 73.5% | 65457 | 2 | Circle | 3.43 | | 1.21% |
| Example 5 | Hex45-50 | 45.9 | 8.70 | 45.8 | 5.26 | 70.7% | 38732 | 2 | Rectangle with rounded corners | 3.15 | 5.19 | 1.27% |
| Example 6 | Hex50-50 | 51.3 | 9.00 | 49.9 | 5.54 | 72.4% | 31756 | 2 | Rectangle with rounded corners | 2.90 | 4.65 | 0.86% |
| Example 7 | Hex50-50 | 51.3 | 9.00 | 49.9 | 5.54 | 72.4% | 31756 | 3 | Circle | 2.80 | | 0.59% |
| Example 8 | Hex50-50 | 51.3 | 9.00 | 49.9 | 5.54 | 72.4% | 31756 | 7 | Circle | 2.84 | | 1.41% |
| Comparative Example | Hex50-50 | 51.3 | 9.00 | 49.9 | 5.54 | 72.4% | 31756 | 2 | Circle | 2.76 | | 0.38% |

As shown in Table 1, although the total opening ratio of the through holes 8 was 0.50% or more in Examples 1 to 8, the total opening ratio does not reach 0.50% and was 0.38% in the Comparative Example.

### [Experiment 4]

A liquid passage time of ethyl alcohol was measured using the well array filters of Examples 2, 6, 7, and 8 and the Comparative Example. Each of the well array filters was installed in a particle alignment device 40 as shown in FIGS. 12 and 13. The well formation region of the well array filter was a square of 17 mm×17 mm. 0.5 ml of ethyl alcohol was injected into the second chamber 48 and a liquid passage time (seconds) until the entire amount of ethyl alcohol passed through the well array filter and flowed into the flow path 56 was measured. The results are shown in Table 2.

**TABLE 2**

| | | Through hole shape | | | | | Ethanol passage time (s) |
|---|---|---|---|---|---|---|---|
| | | Number of through holes | Opening shape | Opening dimension (µm) | | Opening ratio | |
| Example 2 | Hex20-25 | 2 | Circle | 2.80 | | 1.59% | 15 |
| Example 6 | Hex50-50 | 2 | Rectangle with rounded corners | 2.90 | 4.65 | 0.86% | 24 |
| Example 7 | Hex50-50 | 3 | Circle | 2.80 | | 0.59% | 51 |
| Example 8 | Hex50-50 | 7 | Circle | 2.84 | | 1.41% | 22 |
| Comparative Example | Hex50-50 | 2 | Circle | 2.76 | | 0.38% | 99 |

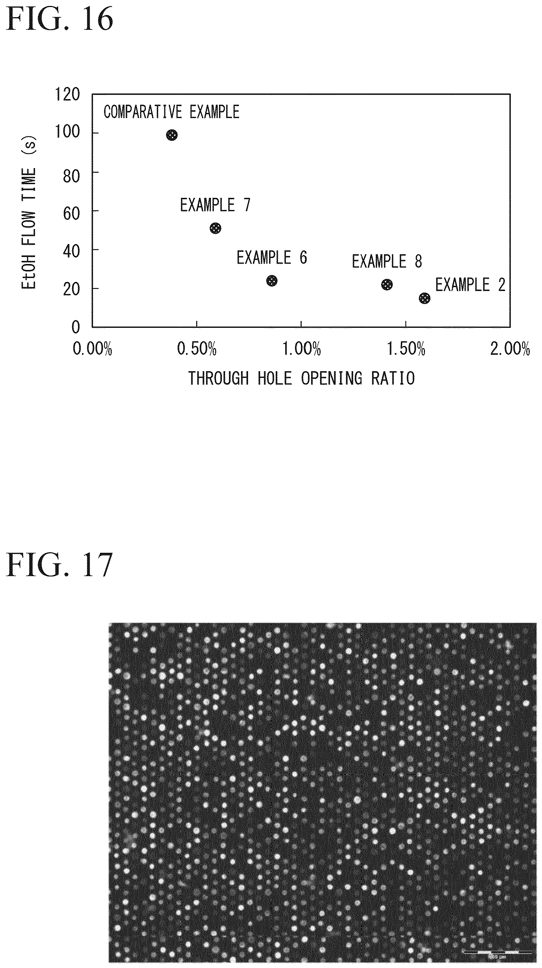

In Examples 2, 6, 7, and 8, the liquid passage was completed within 51 seconds, whereas in the Comparative Example, it took 99 seconds to pass the liquid. In practical terms, it can be seen that a through hole opening ratio of 0.5% or more is desirable because a liquid passage time of ethyl alcohol of 60 seconds of less allows ease of use. FIG. 16 is a graph plotting the results of Table 2.

### [Experiment 5]

The well array filter of Example 2 (well formation region: 17 mm×17 mm) was attached to the particle alignment device 40, and after ethyl alcohol was passed through, a phosphate buffer solution (PBS) was passed through three times. Subsequently, 1 ml of a phosphate buffer solution in which various types of cells were dispersed in amounts equal to the number of seeding cells was introduced into the second chamber 48 and passed through the well array filter. Subsequently, the cells in the wells 3 were stained using a calcein aqueous solution and the cells in the wells 3 were observed through the bottom plate portion 42 using an inverted fluorescence microscope. In addition, the single cell rate (%) was calculated by dividing the number of wells 3 in which only one cell C was captured in the field of view of the microscope by the total number of wells 3.

As the cells, PBMC (peripheral blood mononuclear cells), PC3 (prostate cancer cells), HL60 (leukemia cells), MCF7 (breast cancer cells), and A549 (human alveolar basal adenocarcinoma cells) were used. The number of cells seeded on the well array filter was 10,000, 100,000, and 200,000. The results are shown in Table 3.

**TABLE 3**

| Number of cells seeded | Number of cells/number of wells (%) | Single cell rate (%) | | | | | |
|---|---|---|---|---|---|---|---|
| | | PBMC | PC3 | HL60 | MCF7 | A549 | Poisson distribution |
| 10000 | 2.70 | 94.4 | 100 | 100 | 100 | 100 | 98.7 |
| 10000 | 27.03 | 92.9 | 100 | 99.3 | 98.4 | 98.2 | 87.1 |
| 20000 | 54.05 | 82.6 | 100 | 98.8 | 98.7 | 99.6 | 75.4 |

"Poisson distribution" values in Table 3 indicate a single cell rate (%) which is statistically calculated from a ratio of the number of seeded cells to the number of wells. As shown in Table 3, also for all five types of cells, a single cell rate higher than the single cell rate of the Poisson distribution obtained by statistical probability calculation was obtained. This indicates that, after the first cells are captured in the wells 3, it becomes difficult for the second cells to enter and indicates that the shape of the wells 3 and the setting of the through hole 8 are appropriate.

Particularly, in cancer cell lines (PC3, HL60, MCF7, A549) in which the size of the cells was about 10 mm and the grain diameter was large compared to the opening diameter of the through hole 8, a high single cell rate was obtained regardless of the ratio of the number of cells/the number of wells.

FIG. 17 is a fluorescent micrograph (magnification: 10x) of the cells in the wells 3 of the well array filter after seeding with 200,000 A549 cells and staining with calcein. As shown in FIG. 17, all the A549 cells were regularly stored in the wells 3 and the A549 cells remaining on the well partition walls 6 were not found.

### [Experiment 6]

The well array filters of Example 2 and Example 6 were installed in the particle alignment device 40 and the A549 cells dispersed in a phosphate buffer solution were seeded thereon. In addition, the A549 cells were stored as single cells in the well 3. Subsequently, an automated cell pickup device, manufactured by ALS Automated Lab Solutions under the trade name "CellCelector," was used and the lower end of a glass capillary with an inner diameter of 20 µm was placed vertically directly above a predetermined well 3 in which cells were stored, and 100 nL of liquid was aspirated from inside the well 3. Through aspiration, the cells stored in the wells 3 surrounding the predetermined wells 3 were not suctioned up and only the cells stored in the predetermined wells 3 could be collected using the glass capillary. The tip of the glass capillary was not damaged.

### INDUSTRIAL APPLICABILITY

According to the present invention, since the well partition walls between the wells are sufficiently thin, particles such as cells and beads are less likely to remain on the well partition walls. In addition, by appropriately setting the opening diameter, a percentage at which excessive particles enter one of the wells can be reduced and the particles can be stably captured in the wells which have a sufficient depth compared to the opening diameter. Furthermore, since the wells have a polygonal shape, the wells are easily distinguished from particles during automated image analysis, and furthermore, since the wells can be arranged at high density, excellent effects in which a number of particles can be screened at once are achieved. Therefore, the present invention is industrially applicable.

### REFERENCE SIGNS LIST

1 Well array filter
2 Filter body
3 Well
4 Well bottom portion
4A Bottom portion resist
6 Well partition wall
6A Partition wall resist
8 Through hole
10 Substrate
12 Sacrificial film
14 Gap
20 Well array filter
30 Well array filter
40 Particle alignment device
41 Device body
42 Bottom plate portion
44 Wall portion
46 First chamber
48 Second chamber
50 Third chamber
52 Opening
54 Opening
56 Flow path
A Opening diameter
B Partition wall thickness
C Cell
P Pipette
W Interval
D Diameter

## Claims

1. A well array filter, comprising a flat filter body,
wherein the filter body has a plurality of wells each opening in a well formation region on an upper surface of the filter body,
the wells which are adjacent to each other are separated by a well partition wall,
a well bottom portion is formed at a lower end of each of the wells,
an opening of each of the wells has a polygonal shape or a polygonal shape with rounded corners,
an equivalent circle diameter of the opening of each of the wells is 5 µm or more and 200 µm or less,
at least one through hole is formed in the well bottom portion to reach a lower surface of the filter body,
a maximum thickness in a horizontal direction of each of the well partition walls is 1 µm or more and 20 µm or less,
a ratio of a total opening area of the well to an area of the well formation region is 40% or more, and
a depth of each of the wells is at least twice the maximum thickness in the horizontal direction of the well partition wall.

2. The well array filter according to claim 1, wherein a maximum inscribed circle diameter of the through hole is 4 µm or less.

3. The well array filter according to claim 1 or 2, wherein a ratio of a total opening area of the through holes to the area of the well formation region is 0.5% or more and 3.5% or less.

4. The well array filter according to claim 1 or 2, wherein the polygonal shape is any of a triangular shape, a quadrangular shape, and a sexangular shape.

5. The well array filter according to claim 1 or 2, wherein the well partition wall and the well bottom portion are formed of different materials and the well bottom portion is formed of a material stronger than that of the well partition wall.

6. The well array filter according to claim 1 or 2, wherein the well partition wall and the well bottom portion are formed of different materials, each of the well bottom portions is divided in a tile shape corresponding to a shape of the opening of the well, gaps are formed between neighboring well bottom portions, and a material forming the well partition walls is filled and solidified in the gaps.

7. The well array filter according to claim 1 or 2, wherein at least an inner surface of the well is coated with a polymer which inhibits adhesion of cells.

8. The well array filter according to claim 1 or 2, wherein a thickness of the well bottom portion is 1 nm or more and 2 µm or less.

9. A particle alignment device, comprising:
the well array filter according to claim 1 or 2; and
a device body that supports the well array filter and has a sample flow path extending from a well opening side of the well array filter through the through holes toward bottom sides of the wells.

10. A particle capturing method, comprising:
a step of passing a liquid including an organic solvent which does not dissolve or degrade the well array filter and the device body through the sample flow path of the particle alignment device according to claim 9;
a step of passing an aqueous solution through the sample flow path and replacing the liquid including the organic solvent with the aqueous solution; and
a step of passing an aqueous solution including particles to be captured in the wells through the sample flow path and capturing the particles in the wells.
